# EUROPEAN PATENT SPECIFICATION

(11) **EP 4 137 123 B1**
(45) Date of publication and mention of the grant of the patent: **30.09.2026**
(21) Application number: 22187779.8
(22) Date of filing: 21.12.2018
(51) Int. Cl.: A61K 9/127, A61K 31/4196

(54) **INJECTABLE COMPOSITIONS OF TRITERPENOID ANTIFUNGALS ENCAPSULATED IN LIPOSOMES**
INJIZIERBARE ZUSAMMENSETZUNGEN VON IN LIPOSOMEN EINGEKAPSELTEN TRITERPENOID-ANTIMYKOTIKA
COMPOSITIONS INJECTABLES D'ANTIFONGIQUES TRITERPÉNOÏDES ENCAPSULÉES DANS DES LIPOSOMES

(30) Priority: 02.01.2018 US 201862612893 P
(43) Date of publication of application: 22.02.2023
(62) Document number(s) of the earlier application(s) in accordance with Art. 76 EPC: 18834263.8 / 3 735 226
(73) Proprietor: Scynexis, Inc., Jersey City, NJ 07302 (US)
(72) Inventor: MOTHERAM, Rajeshwar, New Jersey 08810 (US)
(74) Representative: Harris, Jennifer Lucy

(56) References cited:
- STEPHEN A. WRING ET AL: "Preclinical Pharmacokinetics and Pharmacodynamic Target of SCY-078, a First-in-Class Orally Active Antifungal Glucan Synthesis Inhibitor, in Murine Models of Disseminated Candidiasis", ANTIMICROBIAL AGENTS AND CHEMOTHERAPY, vol. 61, no. 4, 30 January 2017 (2017-01-30), US, XP055518287, ISSN: 0066-4804, DOI: 10.1128/AAC.02068-16

## Description

### FIELD OF THE INVENTION

The present invention relates to compositions comprising liposome vesicles encapsulating enfumafungin derived triterpenoid antifungal compounds. More particularly, the invention relates to compositions comprising phospholipid bilayer vesicles encapsulating enfumafungin derived triterpenoids of Formula (Ia) (or pharmaceutically acceptable salts or hydrates thereof) that are inhibitors of (1,3)-β-D-glucan synthesis, that can be used to treat and/or prevent systemic fungal infections. The compositions of the present invention have good local tolerability characteristics and are well-suited for administration by injection.

### BACKGROUND OF THE INVENTION

Fungal infections are a major healthcare problem and are most commonly manifested as invasive fungal disease (*e*.*g*., candidemia, invasive aspergillosis), localized fungal infections (*e*.*g*., pleural empyema and abscess localized in abdomen, brain, lung, etc.) and mucocutaneous infections (*e*.*g*., oral, esophageal and vulvovaginal candidiasis). The type and scope of the infection depends on the virulence factors of the fungal pathogen, the host's defenses, and the anatomic areas involved.

Severe systemic fungal infections are more common in immuno-compromised patients such as patients receiving chemotherapy to treat malignancies, or receiving immunomodulatory agents to treat chronic inflammatory conditions, or suffering from immune deficiencies, either acquired or due to genetic disorders. Despite currently available antifungal therapies, systemic fungal infections are associated with a mortality rate of up to 50%, depending on the pathogen and the underlying condition of the patient.

Enfumafungin is a hemiacetal triterpene glycoside that is produced in fermentations of a *Hormonema spp.* associated with living leaves of *Juniperus communis* (U.S. Pat. No. 5,756,472; Pelaez et al., Systematic and Applied Microbiology, 23:333-343, 2000; Schwartz et al., JACS, 122:4882-4886, 2000; Schwartz, R.E., Expert Opinion on Therapeutic Patents, 11(11):1761-1772, 2001). Enfumafungin is one of the several triterpene glycosides that have *in vitro* antifungal activities. The mode of the antifungal action of enfumafungin and other antifungal triterpenoid glycosides was determined to be the inhibition of fungal cell wall glucan synthesis by their specific action on (1,3)-β-D-glucan synthase (Onishi et al., Antimicrobial Agents and Chemotherapy, 44:368-377, 2000; Pelaez et al., Systematic and Applied Microbiology, 23:333-343, 2000). 1,3-β-D-glucan synthase remains an attractive target for antifungal drug action because it is present in many pathogenic fungi and therefore affords a broad antifungal spectrum. In addition, because there is no mammalian counterpart to (1,3)-β-D-glucan synthase, the enfumafungin derivatives described herein have little or no mechanism-based toxicity. The triterpenoid compound derivatives of enfumafungin used according to this invention have demonstrated activity against fungal isolates of *Candida* spp., including those isolates that are resistant to azoles or other glucan synthase inhibitors (*e*.*g*., lipopeptide agents such as echinocandins), indicating that the biological and molecular target of the enfumafungin derivatives is different from that of other glucan synthase inhibitors.

Various enfumafungin derivatives have been disclosed, *e*.*g*., in International Patent Publication Nos. WO 2007/126900 and WO 2007/127012.

SCY-078 is a representative compound of the enfumafungin derivatives described herein and has activity against a number of *Candida* and *Aspergillus* species, including drug-resistant strains. SCY-078 is a carboxylic acid and alkyl amino ether substituted triterpenoid derivative having the following formula: 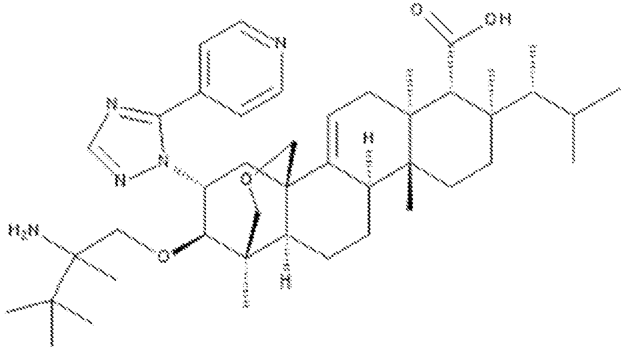 Without intending to be bound by theory: Due to the amino and carboxylic acid functionalities, SCY-078 is an amphiphilic molecule and exhibits surfactant-like behavior including micelle formation, binding to surfaces and plasma proteins. Wring et al., Antimicrobial Agents and Chemotherapy, 61: e02068-16, 2017, disclose pharmacokinetic properties of SCY-078 after i.v. and oral administration.

Intravenous administration of SCY-078 has been associated with local injection site reactions (hereinafter referred to as "ISR"s), including pain, irritation, inflammation, and phlebitis, which has precluded its administration via peripheral veins. While the underlying mechanism of observed ISRs is not clearly understood, and without intending to be bound by theory: The observed ISRs could be attributed to localized direct interaction of SCY-078 with the vascular endothelium at the injection site when SCY-078 is introduced into the bloodstream intravenously.

There is a need in the art to be able to administer SCY-078 and other enfumafungin derivative antifungals intravenously via a peripheral vein with fewer and/or less pronounced ISRs, and to enhance flexibility and convenience for caregivers and patients.

### SUMMARY OF THE INVENTION

The present invention provides injectable compositions as defined in claim 1. The compositions comprise single bilayer vesicles or liposomes encapsulating an enfumafungin derived triterpenoid compound and hydrated in an aqueous phase, suitable for intravenous administration via a peripheral vein. The vesicles may be made of phospholipids and cholesterol. The pH of the aqueous phase is preferably from about 5.0 to about 7.0. A monosaccharide solution and a disaccharide solution are examples of the aqueous phase.

The injectable compositions preferably comprise SCY-078 or a salt thereof as the enfumafungin derivative.

The injectable compositions may be administered intravenously to treat and/or to prevent fungal infections, such as systemic fungal infections, including those caused by *Candida* species or *Aspergillus* species. With the injectable compositions of the present invention, local injection site reactions (including pain, irritation, inflammation, and phlebitis) after intravenous administration can be reduced in number and/or degree as compared with such reactions after intravenous administration of a non-liposomal composition containing the same active.

The present invention also provides the injectable composition of the invention for use in a method of treating or preventing fungal infections in patients by intravenous administration of the injectable composition.

The present invention further provides methods of making compositions according to the invention.

### DETAILED DESCRIPTION OF THE INVENTION

Phospholipids are amphiphilic molecules having a hydrophilic ionizable head or "polar head" and a hydrophobic tail comprising long chain fatty acids. When hydrated, multiple phospholipid molecules (optionally with other molecules such as cholesterol) can form liposomes, which are closed, bilayer concentric vesicles that entrap water or other aqueous medium. The fatty acid tails of the phospholipid molecules form part of the interior of the bilayer membrane of the liposome. The polar heads on one surface of the membrane orient toward the aqueous interior of the liposome and the polar heads on the other surface of the membrane orient toward the aqueous exterior environment.

Depending on their physicochemical characteristics, medications can be incorporated in a liposome either in the aqueous interior or in the lipid bilayer, with hydrophobic molecules primarily being associated in the lipid bilayer and/or hydrophilic molecules being incorporated into (*e*.*g*., dissolved or dispersed in) the aqueous medium in the interior of the liposome.

Liposomes may be unilamellar, that is, having one bilayer of phospholipids enclosing an aqueous center, or multilamellar, that is, comprising many concentric aqueous filled bilayer vesicles. Multilamellar vesicles ("MLV"s) can have a broad and heterogeneous size distribution and generally are not amenable to a reproducible manufacturing process. MLVs are not desirable for intravenous administration due to their large particle size. Further, MLVs are difficult to sterilize by filtration. Unilamellar liposomes that are relatively small are desirable for injectable compositions that are to be administered intravenously.

Although liposomes have been used to deliver medications to target tissues and to reduce systemic toxicity, their usefulness in reducing ISRs occurring locally at an injection or infusion site was not understood clearly. Further, prior to the present invention, it was not known whether incorporation of enfumafungin derived triterpenoids into liposomes would be feasible. Moreover, as to SCY-078 in particular: This drug exhibits a high degree of protein binding, and it was not known whether release or leakage of encapsulated drug would occur upon intravenous administration of SCY-078 into the bloodstream; an effective liposomal composition would need to have liposomes that remain intact in the bloodstream for a sufficient time to minimize release or leakage of encapsulated drug at the infusion site. With the present invention, a composition of phospholipid-cholesterol vesicles encapsulating a enfumafungin derived triterpenoid antifungal such as SCY-078 or a pharmaceutically acceptable salt thereof can be administered intravenously and the vesicles can remain intact in the bloodstream until taken up by macrophages in organs of the reticuloendothelial system (RES) such as the liver, spleen, etc. Still further, though manifestation of ISRs can occur at very low concentrations of free (unencapsulated) SCY-078, with the present invention the severity and frequency of ISRs upon intravenous administration of SCY-078 can be mitigated by minimizing the availability of free drug locally at the injection site. The present invention provides for encapsulation of SCY-078 (or a pharmaceutically acceptable salt thereof) or other enfumafungin derived triterpenoid antifungal in a phospholipid ("PL") based system such as liposomes, as defined in claim 1, as an approach to reducing ISRs resulting from intravenous administration while providing the benefits of the antifungal. With the present invention, SCY-078 can be encapsulated into liposomes with high efficiency to minimize free (unencapsulated) drug and to render the liposomal composition suitable for commercial manufacturing. In addition, the physicochemical characteristics of the liposomes as described herein are stable, and this is conducive to storage of the composition.

The present invention provides an injectable composition comprising:
an aqueous phase; and
one or more unilamellar vesicles that each comprise phospholipid and cholesterol, and that each encapsulate a compound of Formula (Ia) or a pharmaceutically acceptable salt or hydrate thereof: 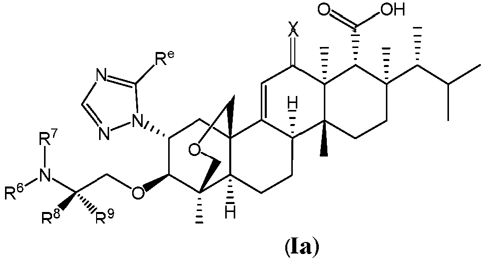 wherein:
   X is O or H, H;
   R^{e} is C(O)NR^{f} R^{g} or a 6-membered ring heteroaryl group containing 1 or 2 nitrogen atoms wherein the heteroaryl group is optionally mono-substituted on a ring carbon with fluoro or chloro or on a ring nitrogen with oxygen;
   R^{f}, R^{g}, R⁶ and R⁷ are each independently hydrogen or C₁-C₃ alkyl;
   R⁸ is C₁-C₄ alkyl, C₃-C₄ cycloalkyl or C₄-C₅ cycloalkyl-alkyl;
   R⁹ is methyl or ethyl; and
   R⁸ and R⁹ are optionally taken together to form a 6-membered saturated ring containing 1 oxygen atom,
wherein the one or more unilamellar vesicles are hydrated in the aqueous phase.

The pH of the aqueous phase is preferably from about 5.0 to about 7.0. A monosaccharide solution and a disaccharide solution are examples of the aqueous phase. The injectable composition may be administered intravenously to treat and/or to prevent fungal infections, such as systemic fungal infections, including those caused by *Candida* species or *Aspergillus* species.

The invention also provides the composition of the invention for use in methods of treating and/or preventing a fungal infection by intravenously administering the injectable composition comprising one or more unilamellar vesicles that each comprise phospholipid and cholesterol, and that each encapsulate the compound of Formula (Ia) or a pharmaceutically acceptable salt or hydrate thereof. Further, the invention provides the use of the vesicles encapsulating the compound of Formula (Ia) or a pharmaceutically acceptable salt or hydrate thereof in the preparation of an injectable medicament for treating or preventing a fungal infection.

In embodiment 1': X is H, H, and the other substituents are as provided for in Formula (Ia).

In embodiment 2': R^{e} is either pyridyl or pyrimidinyl optionally mono-substituted on a ring carbon with fluoro or chloro or on a ring nitrogen with oxygen, and the other substituents are as provided in embodiment 1' or in Formula (Ia).

In embodiment 3': R^{e} is 4-pyridyl and the other substituents are as provided in embodiment 1' or in Formula (Ia).

In embodiment 4': R^{e} is C(O)NH₂ or C(O)NH(C₁-C₃ alkyl) and the other substituents are as provided in embodiment 1' or in Formula (Ia).

In embodiment 5': R⁸ is C₁-C₄ alkyl and R⁹ is methyl; and the other substituents are as provided in embodiment 1', 2', 3', or 4', or in Formula (Ia).

In embodiment 6': R⁸ is t-butyl, R⁹ is methyl; and the other substituents are as provided in embodiment 1', 2', 3', or 4', or in Formula (Ia).

In embodiment 7': R⁶ and R⁷ are each independently hydrogen or methyl and the other substituents are as provided in embodiment 1', 2', 3', 4', 5', or 6', or in Formula (Ia).

In preferred embodiments, the present invention provides an injectable composition comprising:
an aqueous phase; and
one or more unilamellar vesicles that each comprise phospholipid and cholesterol, and that each encapsulate a compound of Formula (IIa) or a pharmaceutically acceptable salt or hydrate thereof: 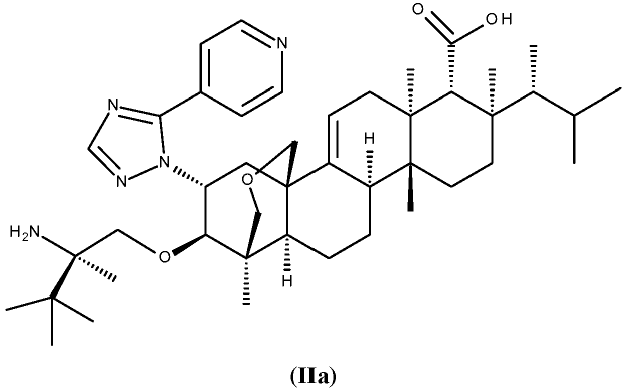 which is (1*S*,4a*R*,6a*S*,7*R*,8*R*,10a*R*,10b*R*,12a*R*,14*R*,15*R*)-15-[[(2*R*)-2-amino-2,3,3-trimethylbutyl]oxy]-8-[(1*R*)-1,2-dimethylpropyl]-14-[5-(4-pyridinyl)-*1H*-1,2,4-triazol-1-yl]-1,6,6a,7,8,9,10,10a,10b,11,12,12a-dodecahydro-1,6a,8,10a-tetramethyl-*4H*-1,4a-propano-*2H-*phenanthro[1,2-c]pyran-7-carboxylic acid,
wherein the one or more unilamellar vesicles are hydrated in the aqueous phase.

The pH of the aqueous phase is preferably from about 5.0 to about 7.0. A monosaccharide solution and a disaccharide solution are examples of the aqueous phase. The injectable composition may be administered intravenously to treat and/or to prevent fungal infections, such as systemic fungal infections, including those caused by *Candida* species or *Aspergillus* species.

The invention also provides the composition of the invention for use in methods of treating and/or preventing a fungal infection by intravenously administering the injectable composition comprising one or more unilamellar vesicles that each comprise phospholipid and cholesterol, and that each encapsulate the compound of Formula (IIa) or a pharmaceutically acceptable salt or hydrate thereof. Further, the invention provides the use of the vesicles encapsulating the compound of Formula (IIa) or a pharmaceutically acceptable salt or hydrate thereof in the preparation of an injectable medicament for treating or preventing a fungal infection.

In preferred embodiments, the phosphate salt of a compound of Formula (Ia), or (IIa) is used or administered as described herein.

In preferred embodiments, the citrate salt of a compound of Formula (Ia), or (IIa) is used or administered as described herein.

The present invention also provides an injectable composition as defined in claim 1 comprising: one or more unilamellar vesicles that encapsulate a compound of Formula (Ia), or (IIa), or a pharmaceutically acceptable salt or hydrate thereof; and a pharmaceutically acceptable carrier, adjuvant, or vehicle, for use in the treatment or prevention of a fungal infection.

In the description of compounds in the embodiments set forth above, indicated substitutions are included only to the extent that the substituents provide stable compounds consistent with the definition.

The compounds of Formula (Ia), and (IIa), and pharmaceutically acceptable salts and/or hydrate forms thereof, have antimicrobial (*e*.*g*., antifungal) activities against yeasts and other fungi, including one or more of *Acremonium*, *Absidia* (*e*.*g*., *Absidia corymbifera*), *Alternaria*, *Aspergillus* (*e*.*g*., *Aspergillus clavatus*, *Aspergillus flavus*, *Aspergillus fumigatus*, *Aspergillus nidulans*, *Aspergillus niger*, *Aspergillus terreus*, and *Aspergillus versicolor*), *Bipolaris, Blastomyces* (*e*.*g*., *Blastomyces dermatitidis*), *Blastoschizomyces* (*e*.*g*., *Blastoschizomyces capitatus*), *Candida* (*e*.*g*., *Candida albicans*, *Candida glabrata*, *Candida guilliermondii*, *Candida kefyr*, *Candida krusei*, *Candida lusitaniae*, *Candida parapsilosis*, *Candida pseudotropicalis*, *Candida stellatoidea*, *Candida tropicalis*, *Candida utilis*, *Candida lipolytica*, *Candida famata* and *Candida rugosa*), *Cladosporium* (*e*.*g*., *Cladosporium carrionii* and *Cladosporium trichloides*), *Coccidioides* (*e*.*g*., *Coccidioides immitis*), *Cryptococcus* (*e*.*g*., *Cryptococcus neoformans*), *Curvularia*, *Cunninghamella* (*e*.*g*., *Cunninghamella elegans*), *Dermatophyte*, *Exophiala* (*e*.*g*., *Exophiala dermatitidis* and *Exophiala spinifera*), *Epidermophyton* (*e*.*g*., *Epidermophyton floccosum*), *Fonsecaea* (*e*.*g*., *Fonsecaea pedrosoi*), *Fusarium* (*e*.*g*., *Fusarium solani*), *Geotrichum* (*e*.*g*., *Geotrichum candidum* and *Geotrichum clavatum*), *Histoplasma* (*e*.*g*., *Histoplasma capsulatum var*. *capsulatum*), *Malassezia* (*e*.*g*., *Malassezia furfur*), *Microsporum* (*e*.*g*., *Microsporum canis and Microsporum gypseum*), *Mucor*, *Paracoccidioides* (*e*.*g*., *Paracoccidioides brasiliensis*), *Penicillium* (*e*.*g*., *Penicillium marneffei*), *Phialophora*, *Pityrosporum ovale*, *Pneumocystis* (*e*.*g*., *Pneumocystis carinii*), *Pseudallescheria* (*e*.*g*., *Pseudallescheria boydii*), *Rhizopus* (*e*.*g*., *Rhizopus microsporus var. rhizopodiformis* and *Rhizopus oryzae*), *Saccharomyces* (*e*.*g*., *Saccharomyces cerevisiae*), *Scedosporium* (*e*.*g*., *Scedosporium apiosperum*), *Scopulariopsis*, *Sporothrix* (*e*.*g*., *Sporothrix schenckii*), *Trichoderma*, *Trichophyton* (*e*.*g*., *Trichophyton mentagrophytes* and *Trichophyton rubrum*), and *Trichosporon* (*e*.*g*., *Trichosporon asahii*, *Trichosporon beigelii* and *Trichosporon cutaneum*). The compounds are not only useful against organisms causing systemic human pathogenic fungal infections, but also are useful against organisms causing superficial fungal infections such as *Trichoderma* spp. and other *Candida* spp. The compounds are particularly effective against *Candida* species and *Aspergillus* species.

In view of their antifungal activity, compounds of Formula (Ia), and (IIa), and pharmaceutically acceptable salts and/or hydrate forms thereof, are useful for the treatment and/or prevention of one or more of a variety of superficial, cutaneous, mucocutaneous, subcutaneous and systemic fungal infections including in vulva, vagina, skin, eye, hair, nail, oral mucosa, gastrointestinal tract, bronchus, lung, pleura, peritoneum, endocardium, brain, meninges, urinary organ, vaginal portion, oral cavity, kidney, heart, external auditory canal, bone, nasal cavity, paranasal cavity, spleen, liver, hypodermal tissue, lymph duct, articulation, muscle, tendon, interstitial plasma cell in lung, blood, and so on.

The compounds of Formula (Ia), and (IIa), and pharmaceutically acceptable salts and/or hydrate forms thereof, are useful for preventing and treating one or more of various infectious diseases, such as vulvovaginal candidiasis (VVC), dermatophytosis (*e*.*g*., trichophytosis, ringworm or tinea infections), paronychia, pityriasis versicolor, erythrasma, intertrigo, fungal diaper rash, candida vulvitis, candida balanitis, otitis externa, candidiasis (cutaneous and mucocutaneous), chronic mucocandidiasis (*e*.*g*., thrush and vaginal candidiasis), cryptococcosis, geotrichosis, trichosporosis, aspergillosis, penicilliosis, fusariosis, zygomycosis, sporotrichosis, chromomycosis, coccidioidomycosis, histoplasmosis, blastomycosis, paracoccidioidomycosis, pseudallescheriosis, mycetoma, fungal keratitis, otomycosis, pneumocystosis, fungal abscess, fungal pleural empyema, and fungemia. The compounds of Formula (Ia), and (IIa), and pharmaceutically acceptable salts and/or hydrate forms thereof, may also be used as prophylactic agents to prevent systemic and topical fungal infections. Use as prophylactic agents may, for example, be appropriate as part of a selective gut decontamination regimen in the prevention of infection in immuno-compromised patients (*e*.*g*., AIDS patients, patients receiving cancer therapy or transplant patients). Prevention of fungal overgrowth during antibiotic treatment may also be desirable in some disease syndromes or iatrogenic states.

The compounds of Formula (Ia), and (IIa), and pharmaceutically acceptable salts and/or hydrate forms thereof, can be made according to the synthesis methods disclosed in U.S. Patent No. 8,188,085

As used herein, the term "alkyl" refers to any linear or branched chain alkyl group having a number of carbon atoms in the specified range. Thus, for example, "C₁₋₆ alkyl" (or "C₁-C₆ alkyl") refers to all of the hexyl alkyl and pentyl alkyl isomers as well as n-, iso-, sec- and t-butyl, n- and isopropyl, ethyl and methyl. As another example, "C₁₋₄ alkyl" refers to n-, iso-, sec- and t-butyl, n- and isopropyl, ethyl and methyl.

The term "cycloalkyl" refers to any cyclic ring of an alkane having a number of carbon atoms in the specified range. Thus, for example, "C₃₋₄ cycloalkyl" (or "C₃-C₄ cycloalkyl") refers to cyclopropyl and cyclobutyl.

The term "cycloalkyl-alkyl" (or equivalently "alkyl-cycloalkyl") as used herein refers to a system that includes an alkyl portion as described above and also includes a cycloalkyl portion as described above. Attachment to a "cycloalkyl-alkyl" (or "alkyl-cycloalkyl") may be through either the cycloalkyl or the alkyl portion. The specified number of carbon atoms in "cycloalkyl-alkyl" systems refers to the total number of carbon atoms in both the alkyl and the cycloalkyl parts. Examples of C₄-C₅ cycloalkyl-alkyl include but are not limited to methylcyclopropyl, dimethylcyclopropyl, methylcyclobutyl, ethylcyclopropyl, cyclopropylmethyl, cyclopropylethyl and cyclobutylmethyl.

The term "halogen" (or "halo") refers to fluorine, chlorine, bromine and iodine (alternatively referred to as fluoro, chloro, bromo, and iodo).

The term "or" as used herein denotes alternatives that may, where appropriate, be combined.

Unless expressly stated to the contrary, all ranges cited herein are inclusive. For example, a heterocyclic ring described as containing from "1 to 4 heteroatoms" means the ring can contain 1, 2, 3, or 4 heteroatoms. It is also to be understood that any range cited herein includes within its scope all of the sub-ranges within that range. Thus, for example, a heterocyclic ring described as containing from "1 to 4 heteroatoms" is intended to include as aspects thereof, heterocyclic rings containing 2 to 4 heteroatoms, 3 or 4 heteroatoms, 1 to 3 heteroatoms, 2 or 3 heteroatoms, 1 or 2 heteroatoms, 1 heteroatom, 2 heteroatoms, and so forth.

Any of the various cycloalkyl and heterocyclic/heteroaryl rings and ring systems defined herein may be attached to the rest of the compound at any ring atom (*i*.*e*., any carbon atom or any heteroatom) provided that a stable compound results. Suitable 5- or 6-membered heteroaromatic rings include, but are not limited to, pyridyl, pyrazinyl, pyrimidinyl, pyridazinyl and triazolyl.

A "stable" compound is a compound that can be prepared and isolated and whose structure and properties remain or can be caused to remain essentially unchanged for a period of time sufficient to allow use of the compound for the purposes described herein (*e*.*g*., therapeutic or prophylactic administration to a subject). Reference to a compound also includes stable complexes of the compound such as a stable hydrate.

As a result of the selection of substituents and substituent patterns, certain of the compounds of Formula (Ia), and (IIa) can have asymmetric centers and can occur as mixtures of stereoisomers, or as individual diastereomers, or enantiomers. Unless otherwise indicated, all isomeric forms of these compounds (and pharmaceutically acceptable salts and/or hydrate forms thereof), whether isolated or in mixtures, are within the scope of the present invention. Also included within the scope of the present invention are tautomeric forms of the compounds as depicted (and pharmaceutically acceptable salts and/or hydrate forms thereof).

When any variable occurs more than one time in any constituent or in Formula (Ia), or (IIa), its definition on each occurrence is independent of its definition at every other occurrence. Also, combinations of substituents and/or variables are permissible only if such combinations result in stable compounds.

The term "substituted" includes mono- and poly-substitution by a named substituent to the extent such single and multiple substitution (including multiple substitution at the same site) is chemically allowed. Unless expressly stated to the contrary, substitution by a named substituent is permitted on any atom in a ring (*e*.*g*., an aryl, a cycloalkyl, a heteroaryl, or a heterocyclyl) provided such ring substitution is chemically allowed and results in a stable compound.

A bond terminated by a wavy line is used herein to signify the point of attachment of a substituent group or partial structure. This usage is illustrated by the following example:

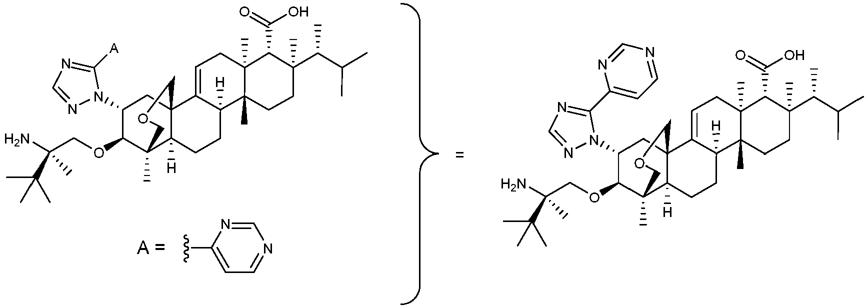

The compounds of Formula (Ia), and (IIa), and pharmaceutically acceptable salts and/or hydrate forms thereof, are also useful in the preparation and execution of screening assays for antifungal compounds. For example, the compounds are useful for isolating mutants, which are excellent screening tools for identifying further antifungal compounds.

The compounds of Formula (Ia), and (IIa) may be administered in the form of "pharmaceutically acceptable salts" or hydrates as appropriate. Other salts may, however, be useful in the preparation of the compounds or of their pharmaceutically acceptable salts. For example, when the compounds contain a basic amine group, they may be conveniently isolated as trifluoroacetic acid salts (*e*.*g*., following HPLC purification). Conversion of the trifluoroacetic acid salts to other salts, including pharmaceutically acceptable salts, may be accomplished by a number of standard methods known in the art. For example, an appropriate ion exchange resin may be employed to generate the desired salt. Alternatively, conversion of a trifluoroacetic acid salt to the parent free amine may be accomplished by standard methods known in the art (*e*.*g*., neutralization with an appropriate inorganic base such as NaHCO₃). Other desired amine salts may then be prepared in a conventional manner by reacting the free base with a suitable organic or inorganic acid. Representative pharmaceutically acceptable quaternary ammonium salts include the following: hydrochloride, sulfate, phosphate, carbonate, acetate, tartrate, citrate, malate, succinate, lactate, stearate, fumarate, hippurate, maleate, gluconate, ascorbate, adipate, gluceptate, glutamate, glucoronate, propionate, benzoate, mesylate, tosylate, oleate, lactobionate, laurylsulfate, besylate, caprylate, isetionate, gentisate, malonate, napsylate, edisylate, pamoate, xinafoate, napadisylate, hydrobromide, nitrate, oxalate, cinnamate, mandelate, undecylenate, and camsylate. Many of the compounds of Formula (Ia), and (IIa) carry an acidic carboxylic acid moiety, in which case suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts.

Also described, but not forming part of the claimed invention, is the use of prodrugs of Formula (Ia), and (IIa). In general, such prodrugs will be functional derivatives of the compounds, which are readily convertible in vivo into the required compound. Thus, in the methods of treatment of the present disclosure the term "administering" shall encompass the treatment of the various conditions described with the compound specifically disclosed or with a compound that converts to the specified compound in vivo after administration to the patient. Conventional procedures for the selection and preparation of suitable prodrug derivatives are described, for example, in "Design of Prodrugs," ed. H. Bundgaard, Elsevier, 1985, which is incorporated by reference herein in its entirety. Metabolites of the compounds of Formula (Ia), and (IIa) include active species produced upon introduction of the compounds into the biological milieu.

The term "administration" and variants thereof (*e*.*g*., "administering" a compound) mean providing a compound to the subject in need of treatment. When a compound of Formula (Ia), and (IIa) or pharmaceutically acceptable salt thereof or a hydrate thereof is provided in combination with a second active agent (*e*.*g*., other antifungal and/or antibacterial agents useful for treating fungal and/or bacterial infections), "administration" and its variants are each understood to include concurrent and sequential provision of the compound (or the salt, or hydrate thereof) and of the other active agent.

As used herein, the term "composition" is intended to encompass a product comprising the specified ingredients, as well as any product that results, directly or indirectly, from combining the specified ingredients.

By "pharmaceutically acceptable" is meant that the ingredients of the pharmaceutical composition must be compatible with each other and not deleterious to the recipient thereof.

The term "subject" (alternatively referred to herein as "patient") as used herein refers to an animal, preferably a mammal, most preferably a human, who has been the object of treatment, observation, or experiment.

The term "effective amount" as used herein means an amount of active ingredient or pharmaceutical agent that elicits the biological or medicinal response in a tissue, system, animal, or human that is being sought by a researcher, veterinarian, medical doctor, or other clinician. In one embodiment, the "effective amount" can be a therapeutically effective amount that alleviates the symptoms of the disease or condition being treated. In another embodiment, the "effective amount" can be a prophylactically effective amount for prophylaxis of the symptoms of the disease or condition being prevented or for reducing the likelihood of occurrence. The term can also refer to an inhibition effective amount of the enfumafungin derivative sufficient to inhibit (1,3)-β-D-glucan synthase and thereby elicit the response being sought.

References to "treat," "treating," "treatment," and variants thereof, generally refer to a treatment that, after it is administered, results in resolution or improvement of one or more signs or symptoms associated with a fungal infection, or that results in eradication of the fungi responsible for an infection, or any combination of these outcomes.

For the purpose of preventing or treating a fungal infection, the compound of Formula (Ia), or (IIa) (optionally in the form of a salt or a hydrate) can be administered in conventional ways available for use in conjunction with pharmaceuticals.

For the purpose of preventing or treating fungal infections that occur in conditions or anatomic areas that have acidic pH, the compound of Formula (Ia), or (IIa) (optionally in the form of a salt or a hydrate) can be administered alone as an individual therapeutic agent or with one or more other antifungal agents (sequentially or concurrently) as a combination of therapeutic agents.

For the purpose of preventing or treating a fungal infection, the compound of Formula (Ia), or (IIa) (optionally in the form of a salt or a hydrate) can be administered with a pharmaceutical carrier selected on the basis of the chosen route of administration and standard pharmaceutical practice.

Further description of methods suitable for use in preparing pharmaceutical compositions and of ingredients suitable for use in said compositions is provided in Remington's Pharmaceutical Sciences, 20th edition, edited by A. R. Gennaro, Mack Publishing Co., 2000.

The compounds of Formula (Ia), and (IIa), and pharmaceutically acceptable salts and/or hydrate forms thereof, can be administered, e.g., intravenously, in a dosage range of, for example, 0.001 to 1000 mg/kg of mammal (*e*.*g*., human) body weight per day in a single dose or in divided doses. An example of a dosage range is 0.01 to 500 mg/kg body weight per day intravenously in a single dose or in divided doses. Another example of a dosage range is 0.1 to 50 mg/kg body weight per day intravenously in single or divided doses. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

Antifungal activity of compounds can be demonstrated by various assays known in the art, for example, by their minimum inhibitory concentration (MIC) against yeasts and minimum effective concentration (MEC) against filamentous molds and dermatophytes in a broth microdilution assay, or in vivo evaluation of the anti-*Candida* and anti-*Aspergillus* activity in mouse or rabbit models. The compounds of Formula (I) provided in the Examples of U.S. Patent No. 8,188,085 were generally found to inhibit the growth of *Candida* spp. in the range of <0.03-32 µg/mL or to give an MEC against*Aspergillus fumigatus* in the range of <0.03-32 µg/mL.

Single bilayer vesicles or liposomes encapsulating enfumafungin derived triterpenoid antifungal compounds according to the present invention preferably have an average particle size (vesicle diameter) of about 150 nm or less, more preferably about 100 nm or less or about 70 to about 80 nm. The vesicles are hydrated in a suitable aqueous phase, such as water containing a monosaccharide or a disaccharide. Any low ionic strength aqueous phase is suitable for hydrating the vesicles. The aqueous phase preferably is isotonic. The pH of the aqueous phase should be between about 4.0 to about 8.0, and preferably from about 5.0 to about 7.0.

Methods for producing single bilayer vesicles or liposomes include methods that provide sufficient energy and shear force (*e*.*g*., sonication, high pressure homogenization, microfluidic mixing, etc.) to form unilamellar vesicles. The single bilayer vesicles described herein can be obtained by sonicating, microfluidically mixing, and/or homogenizing hydrated suspensions comprising an enfumafungin derived antifungal (i.e. a compound of Formula (Ia), or (IIa) or a pharmaceutically acceptable salt or hydrate thereof), phospholipid, and cholesterol. In preferred embodiments, SCY-078 (or a pharmaceutically acceptable salt or hydrate thereof), phospholipid ("PL"), and cholesterol ("CHOL") are suspended in an aqueous phase having a pH of about 5.0 to about 7.0 in a molar ratio of SCY-078 : PL : CHOL of 1 : 7 : 2.5, and subjected to sonication, microfluidic mixing, homogenizing, and/or another process that would result in the formation of single bilayer vesicles comprising phospholipid and cholesterol, encapsulating SCY-078 (or salt or hydrate thereof) inside the vesicles. The citrate salt of SCY-078 is a preferred drug to be encapsulated in the single bilayer vesicles described herein.

A single kind of phospholipid may be used to form single bilayer vesicles as described herein, or two or more different kinds of phospholipids may be used in combination to form the single bilayer vesicles. Phospholipids suitable for use in forming the single bilayer vesicles described herein include but are not limited to synthetically derived or naturally occurring: phosphatidylcholine ("PC"), phosphatidic acid ("PA"), phosphatidylserine ("PS"), phosphatidylethanolamine ("PE"), and phosphatidylglycerol ("PG").

The phospholipids used in forming the single bilayer vesicles described herein are preferably PC and PG used in combination.

Suitable PCs include but are not limited to: synthetically derived dipalmitoyl phosphatidylcholine ("DPPC"), distearoyl phosphatidylcholine ("DSPC"), dilauroyl phosphatidylcholine ("DLPC"), and dimyristoyl phosphatidylcholine ("DMPC"), and PCs from natural sources such as egg PC and soy PC.

Suitable PGs include but are not limited to: synthetically derived dipalmitoyl phosphatidylglycerol ("DPPG"), distearoyl phosphatidylglycerol ("DSPG"), dilauroyl phosphatidylglycerol ("DLPG"), and dimyristoyl phosphatidylglycerol ("DMPG"), and PGs from natural sources such as egg PG.

Preferably, the phospholipid content of vesicles encapsulating enfumafungin derivatives according to the present invention is 50 to 80 mole percent.

Cholesterol stabilizes the lipid bilayer and may prevent leakage of encapsulated drug, but excess amounts of cholesterol can negatively affect encapsulation efficiency. Preferably, the cholesterol content of vesicles encapsulating enfumafungin derivatives according to the present invention is 10 to 30 mole percent.

Preferably, the drug content of vesicles encapsulating enfumafungin derivatives according to the present invention is 5 to 12 mole percent.

The single bilayer vesicles encapsulating an enfumafungin derived triterpenoid antifungal compound are hydrated in an aqueous phase. Preferably, the aqueous phase contains one or more monosaccharides (such as glucose, fructose, and galactose) and/or one or more disaccharides (such as sucrose, trehalose, and lactose), which render the injectable compositions (single bilayer vesicles encapsulating drug, hydrated in the aqueous phase) isotonic and may improve bilayer stability. When monosaccharide(s) are employed in the aqueous phase, they are preferably present in an amount of about 4 to 6% (w/v) relative to the injectable composition (vesicles encapsulating drug, hydrated in the aqueous phase). When disaccharides are employed in the aqueous phase, they are preferably present in an amount of about 8 to 10% (w/v) relative to the injectable composition (vesicles encapsulating drug, hydrated in the aqueous phase).

Preferably, the concentration of encapsulated drug expressed as mg per mL of the injectable composition (single bilayer vesicles encapsulating the drug, hydrated in the aqueous phase) is 0.01 - 50 mg/mL, and more preferably 0.1 - 5 mg/mL.

To prepare a lipid dispersion containing an enfumafungin derived antifungal agent, phospholipid and cholesterol are dissolved in an organic solvent such as a C₁-C₅ alcohol (preferably methanol or ethanol), and the foregoing solution is added to a solution of enfumafungin derived antifungal in an organic solvent such as ethanol. Alternatively, the antifungal agent can be added directly to a solution of phospholipid and cholesterol. In a suitable reaction vessel such as a round bottom flask, the solvent is evaporated (optionally under vacuum) to obtain a lipid dispersion containing the antifungal agent. The solvent may be removed by other methods, such as by using a spray dryer. Advantageously, the lipid dispersion containing the enfumafungin derived antifungal agent is stable and allows for storage and for convenience in that the lipid dispersion can be hydrated at any desired time and carried through other processing to form liposomes.

The lipid dispersion containing the antifungal agent is hydrated in an aqueous solution preferably containing a monosaccharide or a disaccharide to produce a hydrated suspension. The hydrated suspension is mixed using a high-shear mixer (*e*.*g*., at about 10000 rpm) and the resultant multilamellar vesicles are subjected to high pressure homogenization (*e*.*g*., at about 10000 to about 30000 psi, and at a processing temperature in the range of, *e*.*g*., about 25 °C to about 70 °C) to form single bilayer (unilamellar) vesicles. The single bilayer vesicles may be sterilized, such as by passing them through a 0.45 µm and 0.22 µm filter, and/or by moist heat sterilization. The filtered suspension of single bilayer vesicles can be frozen and dehydrated under high vacuum (lyophilized) for long-term storage. Ready-to-use injectable compositions are obtained by adding sterile water to the lyophilized powder and mixing.

The amount of antifungal agent encapsulated in vesicles prepared according to the methods described herein may be determined using, for example, a high-pressure liquid chromatography (HPLC) assay. The size of the vesicles may be determined using, for example, light scattering procedures such as dynamic light scattering (DLS).

Single bilayer vesicles encapsulating an enfumafungin derived antifungal agent were administered into animal models, including rats and rabbits, to evaluate local tolerability (ISRs) upon intravenous administration. Clinical and histopathological evaluation of infusion site tissues were conducted to assess local tolerability. Stability of the vesicles under conditions simulating infusion of vesicles into the bloodstream was also determined to demonstrate the usefulness of the injectable compositions described herein.

### EXAMPLES

The following examples serve only to illustrate the invention and its practice. The examples are not to be construed as limitations on the scope of the invention PREPARATION OF LIPOSOMES ENCAPSULATING SCY-078

### Examples A through I

For examples A through I: a phospholipid mixture comprising DSPC and DSPG; cholesterol; and the enfumafungin derivative SCY-078 in varying molar ratios as shown in Table 1 were dissolved in solvent (ethanol at 70 °C) and subjected to microfluidic mixing (NanoAssemblr^{™}) with water in a volume ratio of 1:3 solvent : water at 65 °C at a flow rate of 10 mL/min to form SCY-078-loaded (-encapsulated) single bilayer vesicles or liposomes. The solvent and free (unencapsulated) drug in the liposome suspension were removed by tangential flow filtration ("TFF").

### Examples J through P

Examples J and K were prepared by thin film hydration method. Specified amounts of: phospholipid mixture comprising DSPC and DSPG; cholesterol; and SCY-078 as shown in Table 2 were dissolved in ethanol at 70 °C. In a round bottom flask as a suitable reaction vessel, the solvent was removed by evaporation under vacuum to obtain a lipid dispersion film containing the antifungal.

For Examples L through P, the solvent was removed by spray drying. Specified amounts of: phospholipid mixture comprising DSPC and DSPG; cholesterol; and SCY-078 as shown in Table 2 were dissolved in ethanol at 70 °C and mixed to form a uniform colloidal dispersion. The dispersion was sprayed as a fine mist at 20 mL/min through a 0.7 mm nozzle at an atomization pressure of about 70 psi and dried at about 50 °C. The spray dried powder was collected and dried to a constant weight under vacuum.

For Examples J through P, the lipid dispersion containing antifungal agent obtained either by thin film evaporation or spray drying was hydrated in aqueous solution containing 7.5% (w/v) sucrose. The hydrated suspension was mixed using a high-shear mixer at 10000 rpm for about 5 minutes and the resultant multilamellar vesicles were subjected to high pressure homogenization (Microfluidics^{®}) at a pressure in the range of from about 10000 to about 30000 psi and using a processing temperature in the range of from about 25 °C to about 70 °C to form single bilayer vesicles.

Liposomes prepared in Examples M, O, and P were subjected to sterilization via filtration through a 0.45 µm and 0.22 µm filter. Liposomes in Example M were lyophilized. Liposomes in example N were sterilized by moist heat at 121 °C for 12 minutes. CHARACTERIZATION OF LIPOSOMES ENCAPSULATING SCY-078

Formulations with various DSPC : Cholesterol : DSPG : SCY-078 molar ratios as shown in Table 1 and Table 2 were evaluated with respect to formation of single bilayer vesicles, particle size distribution, surface charge, and amount of encapsulated SCY-078.

After formation of vesicles or liposomes, free (unencapsulated) SCY-078 was removed by TFF (Examples A through I). Where TFF was not used in the manufacturing process, any unencapsulated SCY-078 was separated from encapsulated SCY-078 by dialysis using a 20000 Dalton molecular weight cut off (MWCO) cellulose ester membrane.

SCY-078 encapsulated in liposomes was separated from any unencapsulated SCY-078 by size exclusion chromatography using a Sephadex^{®} G-25 column. The amount of SCY-078 encapsulated in liposomes was determined using an HPLC assay. SCY-078 was separated on a C18 column and detected by ultraviolet (UV) spectroscopy at 210 nm. The particle size of vesicles was determined by DLS (Zetasizer Nano, Malvern Instruments). The surface charge (zeta potential) was determined by laser doppler micro-electrophoresis in a disposable electrophoretic cell.

As shown in Tables 1 and 2, several formulations with specific molar ratios of DSPC : Cholesterol : DSPG : SCY-078 were found to form single bilayer liposome vesicles or liposomes with entrapment efficiencies of greater than 95%. It was surprisingly found that essentially no free (unencapsulated) drug could be detected even though a process of removal of any unencapsulated drug was not part of the manufacturing process (Examples J through O). It was also surprising that the vesicles remained stable after moist heat sterilization, with no change in vesicle size and with >95% of SCY-078 retained within the vesicles (Example N).

**Table 1. Liposome Compositions Prepared by Microfluidic Mixing in NanoAssemblr^{™}**

| | Molar Ratio | Mole % | | | | | |
|---|---|---|---|---|---|---|---|
| **Example** | SCY-078:PG:PC:CHOL | SCY-078 | PL | CHOL | Average Particle Size (nm) | Zeta Potential (Charge, mV) | % SCY-078 Encapsulated |
| A | 1:1:2.5:1.25 | 17.4 | 60.9 | 21.7 | 4527.3 | -26.31 | 49.3 |
| B | 1:0.5:5:2.5 | 11.1 | 61.1 | 27.8 | 4359 | -6.43 | 58.3 |
| C | 1:1.2:3:1.5 | 14.9 | 62.7 | 22.4 | 175.5 | -31.59 | 82.3 |
| D | 1:1:5:2.5 | 10.5 | 63.2 | 26.3 | 173.3 | -37.93 | 81.7 |
| E | 1:1.5:3.75:1.87 | 12.3 | 64.7 | 23 | 89.6 | -39.72 | 98.0 |
| F | 1:1.6:4:2 | 11.6 | 65.1 | 23.3 | 104.7 | -47.05 | 95.7 |
| G | 1:2:5:2.5 | 9.5 | 66.7 | 23.8 | 124.9 | -53.34 | 96.3 |
| H | 1:3:5:2.5 | 8.7 | 69.7 | 21.6 | 146.0 | -59.33 | 98.3 |
| I | 1:2:5:1.25 | 10.8 | 75.7 | 13.5 | 122.0 | -48.19 | 97.7 |

**Table 2. Liposome Compositions Prepared by High Pressure Homogenization in a Microfluidizer**

| | Molar Ratio | Mole % | | | | | |
|---|---|---|---|---|---|---|---|
| **Example** | SCY-078:PG:PC:CHOL | SCY-078 | PL | CHOL | Average Particle Size (nm) | Zeta Potential (Charge, mV) | % SCY-078 Encapsulated |
| J | 1:1.5:3.75:1.87 | 12.3 | 64.7 | 23 | 81.6 | -32.0 | 99 |
| K | 1:2:5:2.5 | 9.5 | 66.7 | 23.8 | 91.2 | -47.2 | 96 |
| L | 1:2:5:2.5 | 9.5 | 66.7 | 23.8 | 95 | -54.0 | 99 |
| M | 1:2:5:2.5 | 9.5 | 66.7 | 23.8 | 98 | - | 99 |
| N | 1:2:5:1.25 | 9.5 | 66.7 | 23.8 | 100 | -55.6 | >95 |
| O | 1:2:5:1.25 | 9.5 | 66.7 | 23.8 | 51.1 | -47.3 | >99 |
| P | 1:2:5:1.25 | 9.5 | 66.7 | 23.8 | 78 | -74.1 | >95 |

### EVALUATION OF PHYSICOCHEMICAL STABILITY OF SPRAY DRIED LIPID DISPERSION CONTAINING SCY-078

The stability of spray dried intermediate (lipid dispersion containing SCY-078) was evaluated at ambient (25 °C / 60% RH) and accelerated (40 °C / 75% RH) storage conditions. As shown in Table 3, the spray dried intermediate was stable with respect to assay of SCY-078 and to formation of degradation products, with no significant changes or trends observed for up to 3 months when stored under ambient (25 °C / 60% RH) and accelerated (40 °C / 75% RH) storage conditions. These results show that the spray dried intermediate can be conveniently stored, *e*.*g*., under ambient conditions prior to hydration and other processing to form liposomes, a commercially desirable attribute.

**Table 3. Stability Evaluation of SCY-078 Spray Dried Dispersion**

| **Test** | | **Initial** | **3 Months** | |
|---|---|---|---|---|
| | | | 25°C/60%RH | 40°C/75%RH |
| **Appearance *(visual)*** | | White fine powder | White powder | White powder |
| **Assay (mg/g) SCY-078 as free base** | | 89.2 | 92.2 | 90.7 |
| **Degradation Products** | | | | |
| | **Individual (specified & unspecified)** ^{A} | No impurities >0.1% | No impurities >0.1% | No impurities >0.1% |
| | **Total** | No impurities >0.1% | No impurities >0.1% | No impurities >0.1% |
| **Water Content (%)** | | 2.3 | 1.8 | 3.1 |

| | | | | |
|---|---|---|---|---|
| A: Report value for degradation products ≥ 0.1% area (except for peaks present at the same level or less than the level in the API) | | | | |

### EVALUATION OF PHYSICOCHEMICAL STABILITY OF LIPOSOMES ENCAPSULATING SCY-078

The stability at refrigeration conditions (2 °C to 8 °C) of a ready-to-dilute suspension of liposomes encapsulating SCY-078 was evaluated. As shown in Table 4, there was no change in SCY-078 assay and average particle size. Additionally, there was no leakage of encapsulated drug on storage, demonstrating strong association of SCY-078 with the lipid bilayer of the liposomes. These results demonstrate stability and the potential for long term stability suitable for various commercial applications for the liposomal compositions described herein.

**Table 4. Stability Evaluation of SCY-078 Liposome Injection (4 mg/mL concentration)**

| Example | Storage Condition | Storage Time | Assay (mg/mL) | Average Particle Size (nm) | % Encapsulated |
|---|---|---|---|---|---|
| N | | Initial | 3.8 | 100 | >95% |
| N | 2 °C - 8 °C | 3 Months | 3.78 | 89.6 | -- |
| O | | Initial | 3.80 | 51.1 | >99% |
| O | 2 °C - 8 °C | 6 Months | 4.00 | 53.4 | >99% |

### EVALUATION OF PHYSIOLOGICAL STABILITY OF LIPOSOMES ENCAPSULATING SCY-078

Stability of vesicles or liposomes encapsulating SCY-078 was evaluated by incubating the liposomes in fresh 50% bovine serum over 24 hours at 37 °C. The liposome fractions were separated from serum components by size exclusion chromatography using a Sephadex^{®} G-25 column. Collected liposome and serum fractions were analyzed for SCY-078 content by HPLC. Results as shown in Table 5 demonstrate that while no SCY-078 was detected in serum protein fractions, essentially all of the nominal content of SCY-078 was recovered from liposome fractions. Considering the high degree of protein binding by SCY-078, these results surprisingly indicated that SCY-078 remained encapsulated in intact liposomes under conditions simulating intravenous administration into the bloodstream.

### EVALUATION OF LOCAL TOLERABILITY OF LIPOSOMES ENCAPSULATING SCY-078 IN ANIMALS

### An Intravenous Infusion Local Tolerance Study in Sprague Dawley Rats

In a 14-day intravenous infusion study in rats, animals were infused with SCY-078 encapsulated in liposome (10 or 40 mg/kg/day), or with SCY-078 in solution (10 or 40 mg/kg/day), or with saline control. The animals were infused via indwelling catheters surgically implanted in the vena cava; as such, evaluation of the infusion site response emphasized histological observations of vascular inflammation. Overall, the incidence and severity of vascular inflammation increased for SCY-078 administered as a solution, compared to vascular inflammation in the saline-treated animals; however, vascular inflammation was not observed in animals administered with SCY-078 encapsulated in liposome (Table 6).

### A Local Intravenous Irritation Study in New Zealand White Rabbits

This study evaluated the local irritation of saline control, 40 mg/kg/dose SCY-078 in solution, and SCY-078 encapsulated in liposome (10 or 40 mg/kg) under twice daily dosing (6 hours ±15 minutes apart). Doses were administered via one hour intravenous infusion at a rate of 20 mL/hour via indwelling catheter to New Zealand White rabbits for five consecutive days.

Twice daily intravenous infusion of SCY-078 administered as a solution formulation at 40 mg/kg/dose over a period of 1 hour resulted in adverse local reactions at the infusion site and surrounding area, manifested as very slight/slight to severe erythema and edema, which led to the unscheduled euthanasia of all animals in this group after only one day of dosing. In contrast, the animals receiving twice daily intravenous infusion of SCY-078 encapsulated in liposome at 10 and 40 mg/kg were able to complete the planned 5-day dosing and the formulation was well tolerated.

The results for local tolerability studies in animals demonstrated that SCY-078 encapsulated in liposomes was better tolerated than was SCY-078 in solution with respect to vascular inflammation and ISRs at the infusion site and may be suitable for intravenous administration via a peripheral vein.

**Table 5. Stability of Liposomes Encapsulating SCY-078 following Incubation in Fetal Bovine Serum at 37 °C**

| Incubation Time in Serum at 37 °C (Hours) | SCY-078 Recovered (%) in Serum Fractions (0-29) | Liposomes Recovered (%) in Serum Fractions (0-29) | SCY-078 Recovered (%) in Liposome Fractions (29-80)¹ |
|---|---|---|---|
| 0 Hours | <LOD | <LOD | 112 |
| 1 Hour | <LOD | <LOD | 90 |
| 2 Hours | <LOD | <LOD | 118 |
| 4 Hours | <LOD | <LOD | 98 |
| 8 Hours | <LOD | <LOD | 91 |
| 24 Hours | <LOD | <LOD | 96 |
| | | Average | 100.83 |

| | | | |
|---|---|---|---|
| ¹ SCY-078 recovery normalized to liposome recovery. Limit of Detection (LOD): SCY = 0.036 µg/mL; Liposomes = 0.017 µg/mL | | | |

**Table 6. Vascular Inflammation at Infusion Site in Sprague Dawley Rats**

| **Group Designation** | | **Saline Control** | **SCY-078 in Solution** | | **SCY-078 Liposome** | |
|---|---|---|---|---|---|---|
| **Dose Level (mg/kg/day)** | | **0** | **10** | **40** | **10** | **40** |

| ***Infusion site*, *cranial to catheter tip*** | | | | | | |
|---|---|---|---|---|---|---|
| Total | | 0/4 | 2/4 | 2/4 | 0/4 | 0/4 |
| | Minimal | 0 | 2 | 1 | 0 | 0 |
| | Mild | 0 | 0 | 1 | 0 | 0 |

| ***Infusion site, catheter tip*** | | | | | | |
|---|---|---|---|---|---|---|
| Total | | 0/4 | 1/4 | 0/4 | 0/4 | 0/4 |
| | Mild | 0 | 1 | 0 | 0 | 0 |

| ***Infusion site, caudal to catheter tip*** | | | | | | |
|---|---|---|---|---|---|---|
| Total | | 0/4 | 0/4 | 0/4 | 0/4 | 0/4 |
| | Minimal | 0 | 0 | 0 | 0 | 0 |

## Claims

1. An injectable composition comprising:
an aqueous phase; and
one or more unilamellar vesicles that each comprise phospholipid and cholesterol, and that each encapsulate a compound of Formula (Ia) or a pharmaceutically acceptable salt or hydrate thereof: 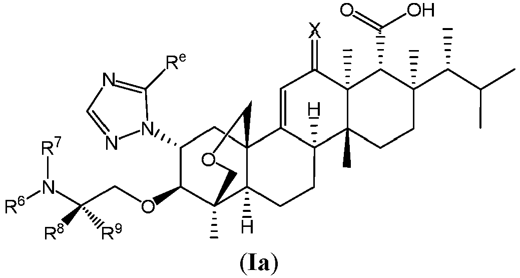 wherein:
X is O or H, H;
R^{e} is C(O)NR^{f}R^{g} or a 6-membered ring heteroaryl group containing 1 or 2 nitrogen atoms wherein the heteroaryl group is optionally mono-substituted on a ring carbon with fluoro or chloro or on a ring nitrogen with oxygen;
R^{f}, R^{g}, R⁶ and R⁷ are each independently hydrogen or C₁-C₃ alkyl;
R⁸ is C₁-C₄ alkyl, C₃-C₄ cycloalkyl or C₄-C₅ cycloalkyl-alkyl;
R⁹ is methyl or ethyl; and
R⁸ and R⁹ are optionally taken together to form a 6-membered saturated ring containing 1 oxygen atom,
wherein the one or more unilamellar vesicles are hydrated in the aqueous phase.

2. The injectable composition according to claim 1, wherein X is H, H.

3. The injectable composition according to claim 1 or 2, wherein R^{e} is:
(a) either pyridyl or pyrimidinyl optionally mono-substituted on a ring carbon with fluoro or chloro or on a ring nitrogen with oxygen, optionally wherein R^{e} is 4-pyridyl; or
(b) C(O)NH₂ or C(O)NH(C₁-C₃ alkyl).

4. The injectable composition according to any one of claims 1 to 3, wherein R⁸ is C₁-C₄ alkyl and R⁹ is methyl, optionally wherein R⁸ is t-butyl and R⁹ is methyl.

5. The injectable composition according to any one of claims 1 to 4, wherein R⁶ and R⁷ are each independently hydrogen or methyl.

6. The injectable composition according to claim 1, wherein the concentration of the encapsulated compound of Formula (Ia) or a pharmaceutically acceptable salt or hydrate thereof in the injectable composition is from about 0.01 to about 50 mg/mL.

7. The injectable composition according to claim 1, wherein:
(a) the aqueous phase comprises sugar, optionally wherein the sugar is selected from monosaccharides, disaccharides, and combinations thereof, optionally wherein the sugar is selected from sucrose, trehalose, lactose, glucose, fructose, and galactose, and combinations thereof; and/or
(b) the pH of the aqueous phase is in the range of from about 5.0 to about 7.0.

8. The injectable composition according to claim 1, wherein the one or more unilamellar vesicles comprise:
(a) phosphatidylcholine, phosphatidic acid, phosphatidylserine, phosphatidylethanolamine, phosphatidylglycerol, or combinations thereof; or
(b) phosphatidylcholine and phosphatidylglycerol, optionally wherein:
(i) the phosphatidylcholine is selected from dipalmitoyl phosphatidylcholine, distearoyl phosphatidylcholine, egg phosphatidylcholine, soy phosphatidylcholine, dilauroyl phosphatidylcholine, and dimyristoyl phosphatidylcholine; and/or
(ii) the phosphatidylglycerol is selected from dipalmitoyl phosphatidylglycerol, distearoyl phosphatidylglycerol, dilauroyl phosphatidylglycerol, and dimyristoyl phosphatidylglycerol.

9. The injectable composition according to claim 1, wherein the compound of Formula (Ia) or a pharmaceutically acceptable salt or hydrate thereof is present in the vesicles in an amount of about 5 to about 12 mole percent, the phospholipid is present in the vesicles in an amount of about 50 to about 80 mole percent, and the cholesterol is present in the vesicles in an amount of about 10 to about 30 mole percent.

10. The injectable composition according to claim 1, wherein the average particle size of the one or more unilamellar vesicles is less than about 150 nm, optionally less than about 100 nm, optionally from about 70 to about 80 nm.

11. The injectable composition according to claim 1, wherein the one or more unilamellar vesicles encapsulate a pharmaceutically acceptable salt of the compound of Formula (Ia), optionally wherein the one or more unilamellar vesicles encapsulate the citrate salt of the compound of Formula (Ia).

12. The injectable composition according to claim 1, for use in a method of treating a fungal infection in a subject in need thereof, the method comprising intravenously administering the injectable composition, optionally wherein the subject is a human.

13. The injectable composition for use according to claim 12, wherein the fungal infection is:
(a) caused by *Candida* spp; or
(b) caused by *Aspergillus* spp; or
(c) is candidemia; or
(d) is invasive aspergillosis.

## Patentansprüche

1. Injizierbare Zusammensetzung umfassend:
eine wässrige Phase; und
ein oder mehrere unilamellare Vesikel, die jeweils Phospholipid und Cholesterol umfassen und die jeweils eine Verbindung der Formel (Ia) oder ein pharmazeutisch annehmbares Salz oder Hydrat davon verkapseln: 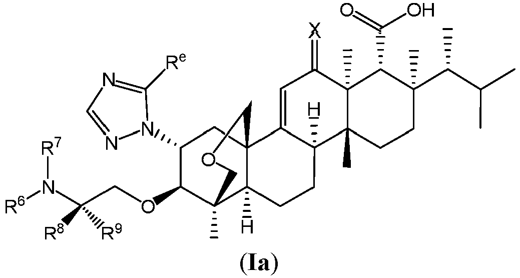 wobei:
X O oder H, H ist;
R^{e} C(O)NR^{f}R^{g} oder eine Heteroarylgruppe mit 6-gliedrigem Ring mit 1 oder 2 Stickstoffatomen ist, wobei die Heteroarylgruppe gegebenenfalls an einem Ringkohlenstoff mit Fluor oder Chlor oder an einem Ringstickstoff mit Sauerstoff monosubstituiert ist;
R^{f}, R^{g}, R⁶ und R⁷ jeweils unabhängig Wasserstoff oder C₁-C₃-Alkyl sind;
R⁸ C₁-C₄-Alkyl, C₃-C₄-Cycloalkyl oder C₄-C₅-Cycloalkylalkyl ist;
R⁹ Methyl oder Ethyl ist; und
R⁸ und R⁹ gegebenenfalls zusammengenommen sind, um einen 6-gliedrigen gesättigten Ring, der 1 Sauerstoffatom enthält, zu bilden,
wobei das eine oder die mehreren unilamellaren Vesikel in der wässrigen Phase hydratisiert sind.

2. Injizierbare Zusammensetzung nach Anspruch 1, wobei X H, H ist.

3. Injizierbare Zusammensetzung nach Anspruch 1 oder 2, wobei R^{e} ist:
(a) entweder Pyridyl oder Pyrimidinyl, gegebenenfalls monosubstituiert an einem Ringkohlenstoff mit Fluor oder Chlor oder an einem Ringstickstoff mit Sauerstoff, gegebenenfalls wobei R^{e} 4-Pyridyl ist; oder
(b) C(O)NH₂ oder C(O)NH(C₁-C₃-Alkyl).

4. Injizierbare Zusammensetzung nach einem der Ansprüche 1 bis 3, wobei R⁸ C₁-C₄-Alkyl ist und R⁹ Methyl ist, wobei R⁸ gegebenenfalls t-Butyl ist und R⁹ Methyl ist.

5. Injizierbare Zusammensetzung nach einem der Ansprüche 1 bis 4, wobei R⁶ und R⁷ jeweils unabhängig Wasserstoff oder Methyl sind.

6. Injizierbare Zusammensetzung nach Anspruch 1, wobei die Konzentration der verkapselten Verbindung der Formel (Ia) oder eines pharmazeutisch annehmbaren Salzes oder Hydrats davon in der injizierbaren Zusammensetzung etwa 0,01 bis etwa 50 mg/ml beträgt.

7. Injizierbare Zusammensetzung nach Anspruch 1, wobei:
(a) die wässrige Phase Zucker umfasst, gegebenenfalls wobei der Zucker ausgewählt ist aus Monosacchariden, Disacchariden und Kombinationen davon, gegebenenfalls wobei der Zucker ausgewählt ist aus Saccharose, Trehalose, Lactose, Glucose, Fructose und Galactose und Kombinationen davon; und/oder
(b) der pH-Wert der wässrigen Phase in dem Bereich von etwa 5,0 bis etwa 7,0 liegt.

8. Injizierbare Zusammensetzung nach Anspruch 1, wobei das eine oder die mehreren unilamellaren Vesikel umfassen:
(a) Phosphatidylcholin, Phosphatidsäure, Phosphatidylserin, Phosphatidylethanolamin, Phosphatidylglycerol oder Kombinationen davon; oder
(b) Phosphatidylcholin und Phosphatidylglycerol, gegebenenfalls wobei:
(i) das Phosphatidylcholin ausgewählt ist aus Dipalmitoylphosphatidylcholin, Distearoylphosphatidylcholin, Ei-Phosphatidylcholin, Soja-Phosphatidylcholin, Dilauroylphosphatidylcholin und Dimyristoylphosphatidylcholin; und/oder
(ii) das Phosphatidylglycerol ausgewählt ist aus Dipalmitoylphosphatidylglycerol, Distearoylphosphatidylglycerol, Dilauroylphosphatidylglycerol und Dimyristoylphosphatidylglycerol.

9. Injizierbare Zusammensetzung nach Anspruch 1, wobei die Verbindung der Formel (Ia) oder ein pharmazeutisch annehmbares Salz oder Hydrat davon in den Vesikeln in einer Menge von etwa 5 bis etwa 12 Molprozent vorhanden ist, das Phospholipid in den Vesikeln in einer Menge von etwa 50 bis etwa 80 Molprozent vorhanden ist und das Cholesterol in den Vesikeln in einer Menge von etwa 10 bis etwa 30 Molprozent vorhanden ist.

10. Injizierbare Zusammensetzung nach Anspruch 1, wobei die mittlere Partikelgröße des einen oder der mehreren unilamellaren Vesikel weniger als etwa 150 nm, gegebenenfalls weniger als etwa 100 nm, gegebenenfalls von etwa 70 bis etwa 80 nm, beträgt.

11. Injizierbare Zusammensetzung nach Anspruch 1, wobei das eine oder die mehreren unilamellaren Vesikel ein pharmazeutisch annehmbares Salz der Verbindung der Formel (Ia) verkapseln, gegebenenfalls wobei das eine oder die mehreren unilamellaren Vesikel das Citratsalz der Verbindung der Formel (Ia) verkapseln.

12. Injizierbare Zusammensetzung nach Anspruch 1 zur Verwendung in einem Verfahren zur Behandlung einer Pilzinfektion bei einem Subjekt mit Bedarf daran, wobei das Verfahren intravenöse Verabreichung der injizierbaren Zusammensetzung umfasst, gegebenenfalls wobei das Subjekt ein Mensch ist.

13. Injizierbare Zusammensetzung zur Verwendung nach Anspruch 12, wobei die Pilzinfektion ist:
(a) von Candida spp. verursacht; oder
(b) von Aspergillus spp. verursacht; oder
(c) Candidämie; oder
(d) eine invasive Aspergillose.

## Revendications

1. Composition injectable, comprenant :
une phase aqueuse ; et
une ou plusieurs vésicules unilamellaires qui comprennent chacune un phospholipide et du cholestérol, et qui encapsulent chacune un composé de formule (Ia) ou un sel ou hydrate pharmaceutiquement acceptable de celui-ci : 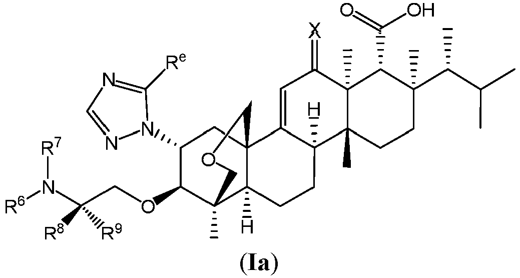
X étant O ou H, H ;
R^{e} étant C(O)NR^{f}R^{g} ou un groupe hétéroaryle à cycle à 6 chaînons contenant 1 ou 2 atomes d'azote, le groupe hétéroaryle étant facultativement mono-substitué sur un carbone de cycle par fluoro ou chloro ou sur un azote de cycle par oxygène ;
R^{f}, R^{g}, R⁶ et R⁷ étant chacun indépendamment hydrogène ou alkyle en C₁-C₃ ;
R⁸ étant C₁-C₄ alkyle, C₃-C₄ cycloalkyle ou C₄-C₅ cycloalkyl-alkyle ;
R⁹ étant méthyle ou éthyle ; et
R⁸ et R⁹ étant éventuellement pris ensemble pour former un cycle saturé à 6 chaînons contenant 1 atome d'oxygène, dans laquelle la ou les vésicules unilamellaires sont hydratées dans la phase aqueuse.

2. Composition injectable selon la revendication 1, X étant H, H.

3. Composition injectable selon la revendication 1 ou 2, dans laquelle R^{e} est :
(a) soit pyridyle, soit pyrimidinyle facultativement mono-substitué sur un carbone de cycle par fluoro ou chloro ou sur un azote de cycle par oxygène, facultativement dans laquelle R^{e} est 4-pyridyle ; ou
(b) C(O)NH₂ ou C(O)NH(alkyle en C₁-C₃).

4. Composition injectable selon l'une quelconque des revendications 1 à 3, dans laquelle R⁸ est C₁-C₄ alkyle et R⁹ est méthyle, facultativement dans laquelle R⁸ est t-butyle et R⁹ est méthyle.

5. Composition injectable selon l'une quelconque des revendications 1 à 4, dans laquelle R⁶ et R⁷ représentent chacun indépendamment hydrogène ou méthyle.

6. Composition injectable selon la revendication 1, dans laquelle la concentration du composé encapsulé de formule (Ia) ou d'un sel ou hydrate pharmaceutiquement acceptable de celui-ci dans la composition injectable est d'environ 0,01 à environ 50 mg/mL.

7. Composition injectable selon la revendication 1, dans laquelle :
(a) la phase aqueuse comprend un sucre, éventuellement dans laquelle le sucre est choisi parmi les monosaccharides, les disaccharides, et leurs combinaisons, éventuellement dans laquelle le sucre est choisi parmi le saccharose, le tréhalose, le lactose, le glucose, le fructose et le galactose, et leurs combinaisons ; et/ou
(b) le pH de la phase aqueuse est dans la plage d'environ 5,0 à environ 7,0.

8. Composition injectable selon la revendication 1, dans laquelle la ou les vésicules unilamellaires comprennent :
(a) phosphatidylcholine, acide phosphatidique, phosphatidylsérine, phosphatidyléthanolamine, phosphatidylglycérol ou leurs combinaisons ; ou
(b) phosphatidylcholine et phosphatidylglycérol, éventuellement dans laquelle :
(i) la phosphatidylcholine est choisie parmi la dipalmitoylphosphatidylcholine, la distéaroylphosphatidylcholine, la phosphatidylcholine d'œuf, la phosphatidylcholine de soja, la dilauroylphosphatidylcholine et la dimyristoylphosphatidylcholine; et/ou
(ii) le phosphatidylglycérol est choisi parmi dipalmitoylphosphatidylglycérol, distéaroylphosphatidylglycérol, dilauroylphosphatidylglycérol et dimyristoylphosphatidylglycérol.

9. Composition injectable selon la revendication 1, dans laquelle le composé de formule (Ia) ou un sel ou hydrate pharmaceutiquement acceptable de celui-ci est présent dans les vésicules en une quantité d'environ 5 à environ 12 pour cent en moles, le phospholipide est présent dans les vésicules en une quantité d'environ 50 à environ 80 pour cent en moles, et le cholestérol est présent dans les vésicules en une quantité d'environ 10 à environ 30 moles pour cent.

10. Composition injectable selon la revendication 1, dans laquelle la taille moyenne de particule de la ou des vésicules unilamellaires est inférieure à environ 150 nm, facultativement inférieure à environ 100 nm, facultativement d'environ 70 à environ 80 nm.

11. Composition injectable selon la revendication 1, dans laquelle la ou les vésicules unilamellaires encapsulent un sel pharmaceutiquement acceptable du composé de formule (Ia), facultativement dans laquelle la ou les vésicules unilamellaires encapsulent le sel de citrate du composé de formule (Ia).

12. Composition injectable selon la revendication 1, pour utilisation dans procédé de traitement d'une infection fongique chez un sujet en ayant besoin, le procédé comprenant l'administration par voie intraveineuse de la composition injectable, facultativement dans laquelle le sujet est un humain.

13. Composition injectable pour une utilisation selon la revendication 12, dans laquelle l'infection fongique est :
(a) causée par Candida spp ; ou
(b) causée par Aspergillus spp ; ou
(c) est la candidémie ; ou
(d) est l'aspergillose invasive.
